# EUROPEAN PATENT APPLICATION

(11) **EP 3 662 908 A1**
(43) Date of publication of application: **10.06.2020**
(21) Application number: 18382890.4
(22) Date of filing: 04.12.2018
(51) Int. Cl.: A61K 31/4412, A61P 9/10

(54) **MODULATING COMPOUNDS OF KCHIP2 AND ITS USE FOR THE TREATMENT OF CARDIOVASCULAR PATHOLOGIES**

(71) Applicant: Consejo Superior de Investigaciones Científicas (CSIC), 28006 Madrid (ES); Universidad Autónoma de Madrid, 28049 Madrid (ES)
(72) Inventor: GUTIERREZ RODRIGUEZ, Marta, 28006 Madrid (ES); VALENZUELA MIRANDA, Carmen, 28029 Madrid (ES); MARTIN MARTINEZ, Mercedes, 28006 Madrid (ES); DELGADO CANENCIA, Carmen, 28029 Madrid (ES); NARANJO OROVIO, Jose Ramón, 28049 Madrid (ES)
(74) Representative: Pons

(57) **Abstract**

The present invention relates to a family of compounds which are capable of modulating KChIP proteins and are therefore useful for the treatment of heart diseases in which the expression levels of this protein are abnormal. These compounds have the following general formula:

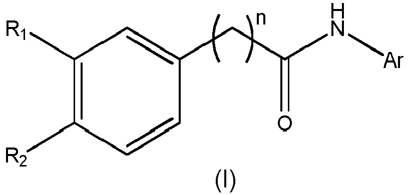

## Description

The present invention relates to a family of compounds which are capable of modulating KChIP2 proteins. Therefore, the invention could fall within the field of pharmacology.

### STATE OF THE ART

KChIPs are accessory proteins which interact with voltage-dependent potassium channels (K_{V}) and belong to the superfamily of neuronal calcium sensors. To date, four isoforms, KChIP1-4, have been described. They all contain a variable N-terminal domain and a C-terminal domain with four EF-hand Ca²⁺-binding domains. In KChIP1-3, the C-terminal region is highly conserved. These three isoforms share a common mechanism of action regarding the modification of the K_{V}4 potassium channel: they increase the potassium current density, they slow down channel inactivation kinetics and speed up the recovery from inactivation. K_{V}4.3 channels generate two currents, depending on the tissue in which they are expressed: a) *I*ₜₒ in the heart and b) A-type currents in the brain. In both cases, the recapitulation of the electrophysiological characteristics is only achieved when channels K_{V}4.3 are expressed together with KChIP subunits. Current *I*ₜₒ is essential for the control of cardiac excitability and is primarily mediated by K_{V}4.3+KChIP2 complexes, whereas *I*_{SA} current participates in the control of neuronal transmission and is mediated by K_{V}4.3+KChIP3 and other KChIPs (1-4). All the isoforms are expressed in the brain, whereas in the heart, KChIP2 is the isoform that is mainly expressed, where it regulates both the current generated by the activation of the K_{V}4.3 channels and the current generated by the Ca_{V}1.2 channels. Various heart diseases, such as cardiac hypertrophy, present a decreased *I*ₜₒ. Moreover, it has been described that mice lacking KChIP2 are more susceptible to generate ventricular arrhythmias.

Taking into account the processes in which the KChIP2 protein is involved, it would be extremely useful to have compounds able to modulate this protein in order to treat diseases in which KChIP2 expression is altered, for example the treatment of heart diseases such as cardiac hypertrophy and heart failure (Grubb et al. Front Physiol. 2012; 3: 118).

Patent document US2008/0039442 describes a series of compounds which interact with the K_{V}4.3 potassium channel for the treatment of heart diseases such as arrhythmia or hypertension, as well as nervous system diseases, such as epilepsy. Patent document WO2008/135447 describes a series of benzamides as BK channel and chloride channel modulators. None of these documents mentions KChIP2 protein.

### DESCRIPTION OF THE INVENTION

The present invention relates to series of compounds having the capacity to modulate KChIP2 function and to affect the transient outward K⁺ current in cardiomyocytes, as demonstrated in the examples. Taking into account that the down-regulation of KChIP2 is associated with the onset of various heart diseases and that the up-regulation of KChIP2 prevents the development of cardiac hypertrophy in animal models, the compounds of the invention, which act as modulators of this protein, are useful in the treatment of heart diseases presenting a decrease in the transient outward K⁺ current.

In a first aspect, the invention relates to a compound of formula (I): 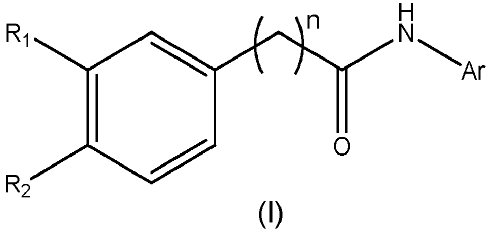 wherein:
R₁ and R₂ are independently selected from H, OH, -O-aryl, halogen, aryl or heteroaryl, wherein said aryl or heteroaryl groups can be optionally substituted with (C₁-C₆) alkyl, - OH, (C₃-C₆) cycloalkyl, -O-(C₁-C₆) alkyl or halogen;
n has a value of 1 or 2;
Ar is selected from the following groups: 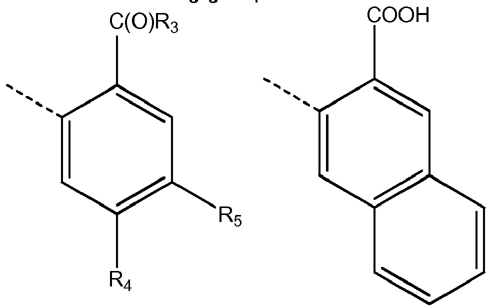 wherein R₃ is selected from -OH or -O-(C₁-C₆)-alkyl; R₄ is selected from H, halogen, -O-(C₁-C₆)-alkyl, phenyl optionally substituted with (C₁-C₆) alkyl; R₅ is selected from H or phenyl optionally substituted with (C₁-C₆) alkyl,
or the salts, isomers or solvates thereof, for use in the treatment of heart diseases such as heart arrhythmia, myocardial ischemia, myocardial infarction, cardiac hypertrophy or cardiomyopathy.

In a preferred embodiment, Ar is the following group: 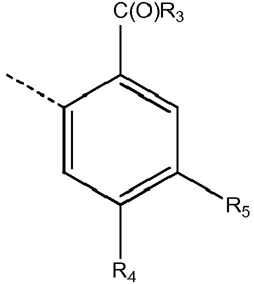 wherein R₃ is OH, R₄ is halogen, preferably Br, and R₅ is H.

In a more preferred embodiment, the compound of formula (I) is 4-bromo-2-[2-(3,4-dichlorophenyl)acetylamino]benzoic acid (32bis).

In a preferred embodiment, the compound of the invention for use as described above is the compound of formula (Ia): 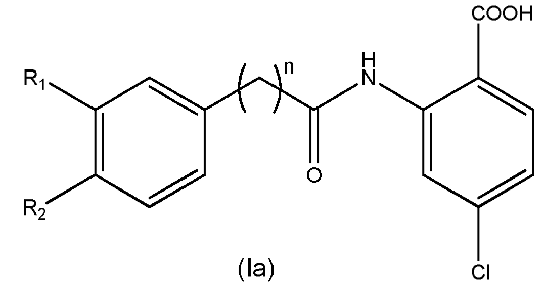 wherein R₁, R₂ and n are defined as above, or the salts, isomers or solvates thereof.

In a more preferred embodiment, in the compound (Ia) for use described above, R₁ is selected from O-phenyl, phenyl, quinoline or pyridine, wherein said phenyl is optionally substituted with a group which is selected from (C₁-C₄) alkyl, -OH or (C₃) cycloalkyl, and said pyridine is optionally substituted with a group which is selected from -O-(C₁-C₄) alkyl, -OH or halogen and R₂ is H.

In an even more preferred embodiment, the compound (Ia) for use as described above is selected from the following list:
- 4-chloro-2-(2-(3-phenoxyphenyl)acetamido)benzoic acid (1)
- 2-(2-([1,1'-biphenyl]-3-yl)acetamido)-4-chlorobenzoic acid (2)
- 2-(2-(4'-butyl-[1,1'-biphenyl]-3-yl)acetamido)-4-chlorobenzoic acid (3)
- 4-chloro-2-(2-(4'-ethyl-[1,1'-biphenyl]-3-yl)acetamido)benzoic acid (4)
- 2-(2-(4'-(*tert*-butyl)-[1,1'-biphenyl]-3-yl)acetamido)-4-chlorobenzoic acid (5)
- 4-chloro-2-(2-(4'-hydroxy-[1,1'-biphenyl]-3-yl)acetamido)benzoic acid (6)
- 4-chloro-2-(2-(4'-(hydroxymethyl)-[1,1'-biphenyl]-3-yl)acetamido)benzoic acid (7)
- 4-chloro-2-(3-(3-phenoxyphenyl)propanamido)benzoic acid (14)
- 2-(3-([1,1'-biphenyl]-3-yl)propanamido)-4-chlorobenzoic acid (15)
- 2-(3-(4'-butyl-[1,1'-biphenyl]-3-yl)propanamido)-4-chlorobenzoic acid (16)
- 4-chloro-2-(3-(4'-ethyl-[1,1'-biphenyl]-3-yl)propanamido)benzoic acid (17)
- 2-(3-(4'-(*tert*-butyl)-[1,1'-biphenyl]-3-yl)propanamido)-4-chlorobenzoic acid (18)
- 4-chloro-2-(3-(4'-(hydroxymethyl)-[1,1'-biphenyl]-3-yl)propanamido)benzoic acid (19)
- 4-chloro-2-(3-(4'-hydroxy-[1,1'-biphenyl]-3-yl)propanamido)benzoic acid (20)
- 4-chloro-2-(2-(4'-cyclopropyl-[1,1'-biphenyl]-3-yl)acetamido)benzoic acid (23)
- 4-chloro-2-(2-(3-(pyridin-2-yl)phenyl)acetamido)benzoic acid (24)
- 4-chloro-2-(2-(3-(quinolin-2-yl)phenyl)acetamido)benzoic acid (25)
- 4-chloro-2-(2-(3-(pyridin-3-yl)phenyl)acetamido)benzoic acid (26)
- 4-chloro-2-(2-(3-(5-fluoropyridin-3-yl)phenyl)acetamido)benzoic acid (27)
- 4-chloro-2-(2-(3-(6-hydroxypyridin-3-yl)phenyl)acetamido)benzoic acid (28)
- 4-chloro-2-(2-(3-(6-ethoxypyridin-3-yl)phenyl)acetamido)benzoic acid (29)
- 4-chloro-2-(3-(3-(pyridin-3-yl)phenyl)propanamido)benzoic acid (30)
- 4-chloro-2-(3-(3-(6-hydroxypyridin-3-yl)phenyl)propanamido)benzoic acid (31)
- 4-chloro-2-(2-(3-(6-ethoxypyridin-3-yl)phenyl)propanamido)benzoic acid (32)
- 4-chloro-2-(3-(3-(5-fluoropyridin-3-yl)phenyl)propanamido)benzoic acid (33)
- 4-chloro-2-[2-(3-phenoxyphenyl)acetylamino]benzoic acid (9bis)
- 4-chloro-2-(2-(3',4'-dichloro-[1,1'-biphenyl]-3-yl) acetylamino)benzoic acid (47bis)

In another more preferred embodiment, in the compound (Ia) for use as described above, R₁ is H and R₂ is selected from O-phenyl, phenyl, quinoline or pyridine, wherein said phenyl is optionally substituted with a group which is selected from (C₁-C₄) alkyl, -OH or (C₃) cycloalkyl, and said pyridine is optionally substituted with a group which is selected from -O-(C₁-C₄) alkyl, -OH or halogen.

In an even more preferred embodiment, the compound (Ia) for use as described above is selected from the following list:
- 4-chloro-2-[2-(3-phenoxyphenyl)acetylamino]benzoic acid (35)
- 2-(2-([1,1'-biphenyl]-4-yl)acetamido)-4-chlorobenzoic acid (36)
- 2-(2-(4'-butyl-[1,1'-biphenyl]-4-yl)acetamido)-4-chlorobenzoic acid (37)
- 4-chloro-2-(2-(4'-ethyl-[1,1'-biphenyl]-4-yl)acetamido)benzoic acid (38)
- 2-(2-(4'-(*tert*-butyl)-[1,1'-biphenyl]-4-yl)acetamido)-4-chlorobenzoic acid (39)
- 4-chloro-2-(2-(4'-(hydroxymethyl)-[1,1 '-biphenyl]-4-yl)acetamido)benzoic acid (40)
- 4-chloro-2-(2-(4'-hydroxy-[1,1'-biphenyl]-4-yl)acetamido)benzoic acid (41)
- 4-chloro-2-(3-(4-phenoxyphenyl)propanamido)benzoic acid (48)
- 2-(3-([1,1 '-biphenyl]-4-yl)propanamido)-4-chlorobenzoic acid (49)
- 2-(3-(4'-butyl-[1,1'-biphenyl]-4-yl)propanamido)-4-chlorobenzoic acid (50)
- 4-chloro-2-(3-(4'-ethyl-[1,1 '-biphenyl]-4-yl)propanamido)benzoic acid (51)
- 2-(3-(4'-(*tert*-butyl)-[1,1'-biphenyl]-4-yl)propanamido)-4-chlorobenzoic acid (52)
- 4-chloro-2-(3-(4'-(hydroxymethyl)-[1,1 '-biphenyl]-4-yl)propanamido)benzoic acid (53)
- 4-chloro-2-(3-(4'-hydroxy-[1,1'-biphenyl]-4-yl)propanamido)benzoic acid (54)
- 4-chloro-2-(2-(4'-cyclopropyl-[1,1'-biphenyl]-4-yl)acetamido)benzoic acid (57)
- 4-chloro-2-(2-(4-(pyridin-2-yl)phenyl)acetamido)benzoic acid (58)
- 4-chloro-2-(2-(4-(pyridin-3-yl)phenyl)acetamido)benzoic acid (59)
- 4-chloro-2-(2-(4-(6-ethoxypyridin-3-yl)phenyl)acetamido)benzoic acid (60)
- 4-chloro-2-(2-(4-(6-hydroxypyridin-3-yl)phenyl)acetamido)benzoic acid (61)
- 4-chloro-2-(2-(4-(5-fluoropyridin-3-yl)phenyl)acetamido)benzoic acid (62)
- 4-chloro-2-(2-(4-(quinolin-2-yl)phenyl)acetamido)benzoic acid (63)
- 4-chloro-2-(3-(4-(quinolin-2-yl)phenyl)propanamido)benzoic acid (64)
- 4-chloro-2-(3-(4-(pyridin-3-yl)phenyl)propanamido)benzoic acid (65)
- 4-chloro-2-(3-(3-(6-hydroxypyridin-3-yl)phenyl)propanamido)benzoic acid (66)
- 4-chloro-2-(2-(4-(6-ethoxypyridin-3-yl)phenyl)propanamido)benzoic acid (67)
- 4-chloro-2-(3-(4-(5-fluoropyridin-3-yl)phenyl)propanamido)benzoic acid (68)

In another preferred embodiment, in the compound (Ia) for use as described above, R₁ and R₂ are chlorine.

In a more preferred embodiment, the compound (Ia) for use as described above is selected from the following list:
- 4-chloro-2-[2-(3,4-dichlorophenyl)acetylamino]benzoic acid, (5bis)
- 4-chloro-2-[3-(3,4-dichlorophenyl)propanoylamino]benzoic acid, (16bis)

In another preferred embodiment, in the compound (Ia) for use as described above, R₁ and R₂ are -OH.

ln a more preferred embodiment, the compound (Ia) for use as described above is 4-chloro-2-[2-(3,4-dihydroxyphenyl)acetylamino]benzoic acid, (15bis).

In another preferred embodiment, the compound of the invention for use as described above is the compound of formula (Ib): 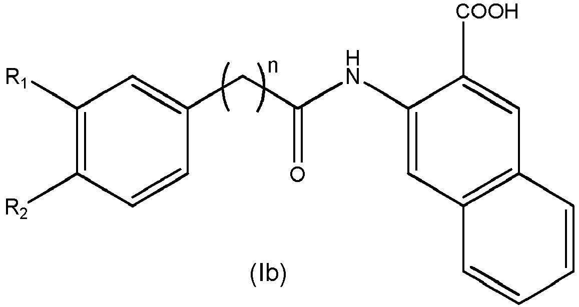 wherein R₁, R₂ and n are defined as in claim 1, or the salts, isomers or solvates thereof.

In a more preferred embodiment, in the compound (Ib) for use described above, R₁ is selected from O-phenyl, chlorine, phenyl, quinoline or pyridine, wherein said phenyl is optionally substituted with a group which is selected from (C₁-C₄) alkyl, -OH or (C₃) cycloalkyl and said pyridine is optionally substituted with a group which is selected from -O-(C₁-C₄) alkyl, -OH or halogen and R₂ is H.

In an even more preferred embodiment, the compound (Ib) for use as described above is selected from the following list:
- 3-(2-([1,1'-biphenyl]-3-yl)acetamido)-2-naphthoic acid (8)
- 3-(2-(4'-butyl-[1,1 '-biphenyl]-3-yl)acetamido)-2-naphthoic acid (9)
- 3-(2-(4'-ethyl-[1,1 '-biphenyl]-3-yl)acetamido)-2-naphthoic acid (10)
- 3-(2-(4'-(*tert*-butyl)-[1,1'-biphenyl]-3-yl)acetamido)-2-naphthoic acid (11)
- 3-(2-(4'-hydroxymethyl-[1,1'-biphenyl]-3-yl)acetamido)-2-naphthoic acid (12) 3-(2-(4'-(hydroxymethyl)-[1,1'-biphenyl]-3-yl)acetamido)-2-naphthoic acid (13)
- 3-(3-([1,1'-biphenyl]-3-yl)propanamido)-2-naphthoic acid (21)
- 3-(2-(4'-cyclopropyl-[1,1'-biphenyl]-3-yl)acetamido)-2-naphthoic acid (22)
- 3-[2-(3-phenoxyphenyl)acetylamino]-2-naphthoic acid (22bis)

In another more preferred embodiment, in the compound (Ib) for use as described above, R₁ is H and R₂ is selected from O-phenyl, chlorine, phenyl, quinoline or pyridine, wherein said phenyl is optionally substituted with a group which is selected from (C₁-C₄) alkyl, -OH or (C₃) cycloalkyl and said pyridine is optionally substituted with a group which is selected from -O-(C₁-C₄) alkyl, -OH or halogen.

In an even more preferred embodiment, the compound (Ib) for use as described above is selected from the following list:
- 3-(2-(4-phenoxyphenyl)acetamido)-2-naphthoic acid (34)
- 3-(2-([1,1'-biphenyl]-4-yl)acetamido)-2-naphthoic acid (42)
- 3-(2-(4'-butyl-[1,1'-biphenyl]-4-yl)acetamido)-2-naphthoic acid (43)
- 3-(2-(4'-ethyl-[1,1'-biphenyl]-4-yl)acetamido)-2-naphthoic acid (44)
- 3-(2-(4'-(*tert*-butyl)-[1,1'-biphenyl]-4-yl)acetamido)-2-naphthoic acid (45)
- 3-(2-(4'-(hydroxymethyl)-[1,1'-biphenyl]-4-yl)acetamido)-2-naphthoic acid (46)
- 3-(2-(4'-hydroxy-[1,1'-biphenyl]-4-yl)acetamido)-2-naphthoic acid (47)
- 3-(3-([1,1'-biphenyl]-4-yl)propanamido)-2-naphthoic acid (55)
- 3-(3-(4'-butyl-[1,1'-biphenyl]-4-yl)propanamido)-2-naphthoic acid (56)

In another more preferred embodiment, in the compound (Ib) for use as described above, R₁ and R₂ are chlorine.

In an even more preferred embodiment, the compound (Ib) for use as described above is selected from the following list:
- 3-[2-(3,4-dichlorophenyl)acetylamino]-2-naphthoic acid, (20bis)
- 3-[3-(3-(3,4-dichlorophenyl)propanoylamino)]-2-naphthoic acid, (23bis)

In another preferred embodiment, the compound of the invention for use as described above is the compound of formula (Ic): 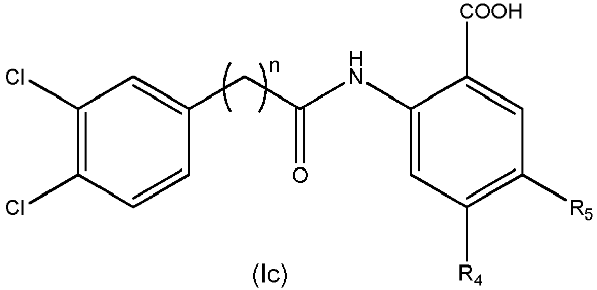 wherein R₄, R₅ and n are defined as above, or the salts, isomers or solvates thereof.

In a more preferred embodiment, in the compound (Ic) for use as described above, R₄ and R₅ are independently selected from H, O-(C₁-C₄) alkyl or phenyl optionally substituted with a (C₁-C₄) alkyl.

In an even more preferred embodiment, the compound (Ic) for use as described above is selected from the following list:
- 2-[2-(3,4-dichlorophenyl)acetylamino]- 4-methoxybenzoic acid (7bis)
- 2-[2-(3,4-dichlorophenyl)acetylamino]-5-(4'-n-butylphenyl)benzoic acid (34bis)
- 2-[2-(3,4-dichlorophenyl)acetylamino]-4-(4'-n-butylphenyl)-benzoic acid (35bis)
- 2-[2-(3,4-dichlorophenyl)acetylamino]-5-(4'-*tert*-butylphenyl)-benzoic acid (36bis)
- 2-[2-(3,4-dichlorophenyl)acetylamino]-5-(2'-methylphenyl)-benzoic acid (37bis)
- 2-[3-(3,4-dichlorophenyl)propanoylamino]-5-(4'-n-butylphenyl)benzoic acid (38bis)
- 2-[2-(3,4-dichlorophenyl)acetylamino]-4-phenylbenzoic acid (39bis)
- 2-[2-(3,4-dichlorophenyl)acetylamino]-4-(2'-methylphenyl)benzoic acid (40bis)
- 2-[3-(3,4-dichlorophenyl)propanoylamino]-4-phenylbenzoic acid (41bis)
- 2-[2-(3,4-dichlorophenyl)acetylamino]-5-phenylbenzoic acid (42bis)
- 2-[3-(3,4-dichlorophenyl)propanoylamino]-5-(4'-*tert*-butylphenyl)benzoic acid (43bis)
- 2-[3-(3,4-dichlorophenyl)propanoylamino]-5-(2'-methylphenyl)benzoic acid (44bis)
- 2-[3-(3,4-dichlorophenyl)propanoylamino]-4-(2'-methylphenyl)benzoic acid (45bis)

| Comp. | Structure |
|---|---|
| 1 | 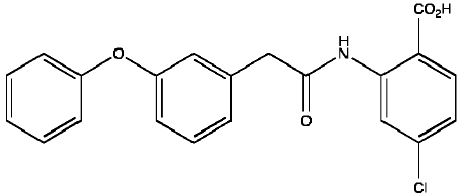 |
| 2 | 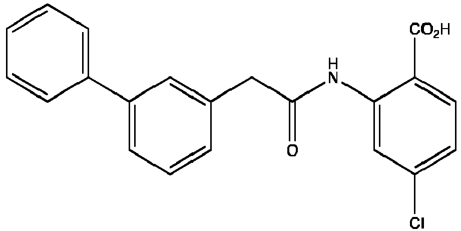 |
| 3 | 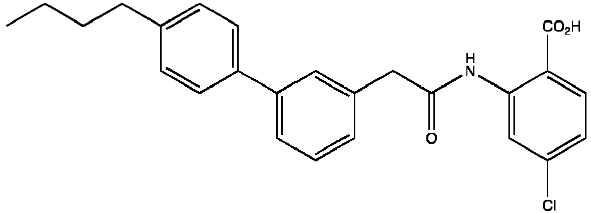 |
| 4 | 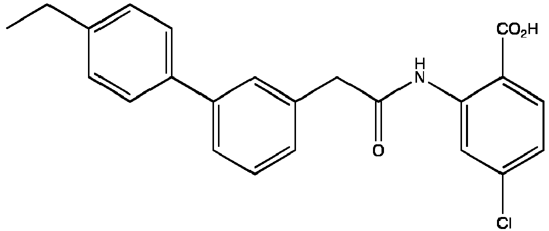 |
| 5 | 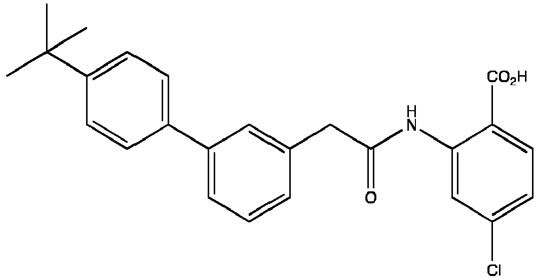 |
| 6 | 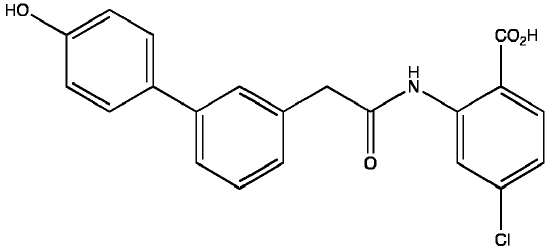 |
| 7 | 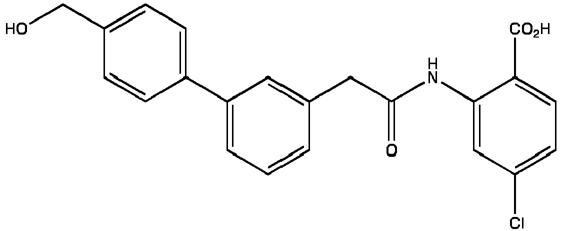 |
| 8 | 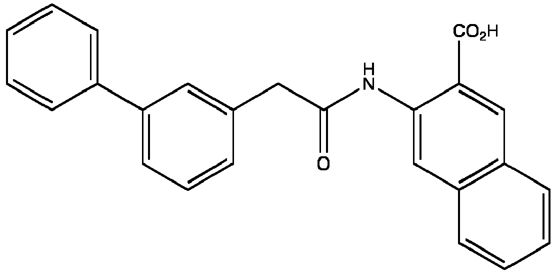 |
| 9 | 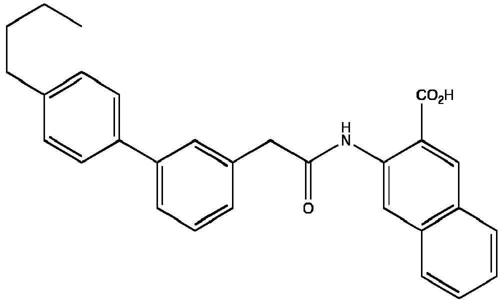 |
| 10 | 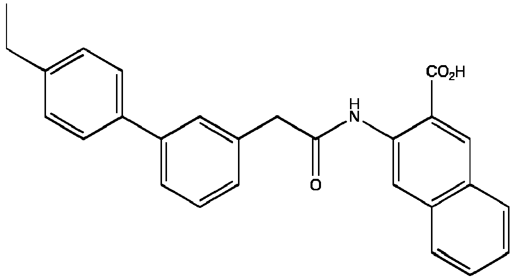 |
| 11 | 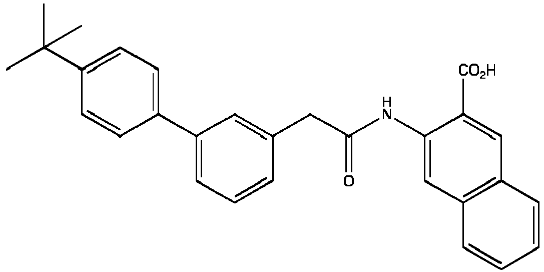 |
| 12 | 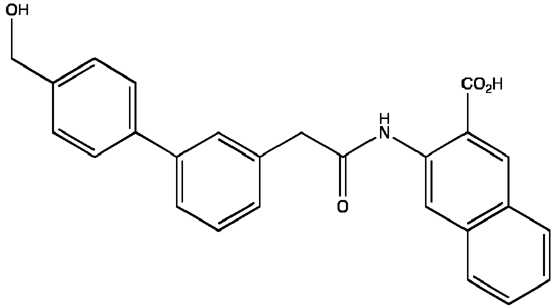 |
| 13 | 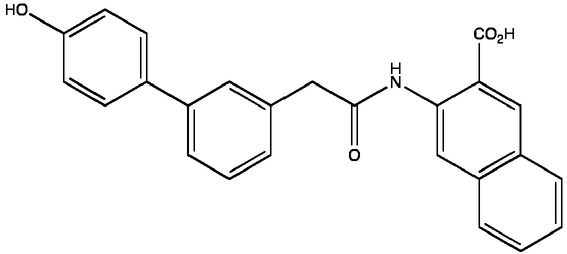 |
| 14 | 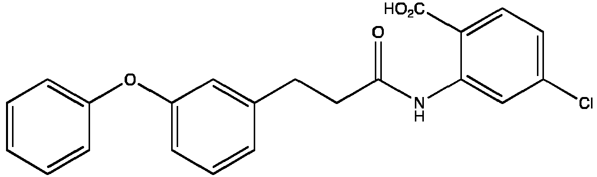 |
| 15 | 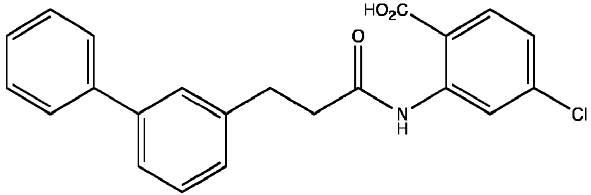 |
| 16 | 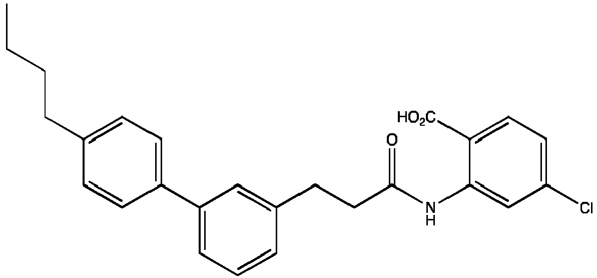 |
| 17 | 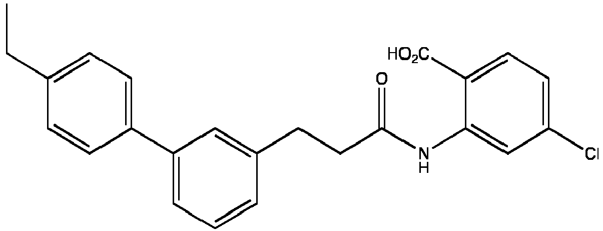 |
| 18 | 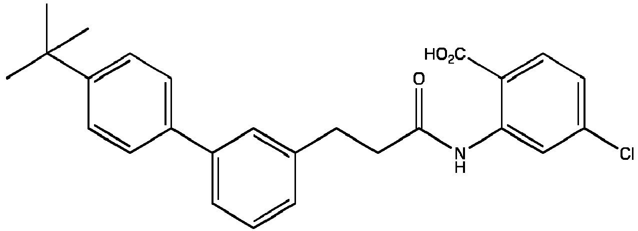 |
| 19 | 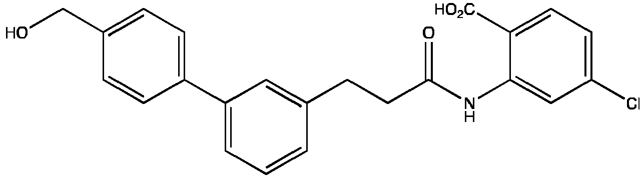 |
| 20 | 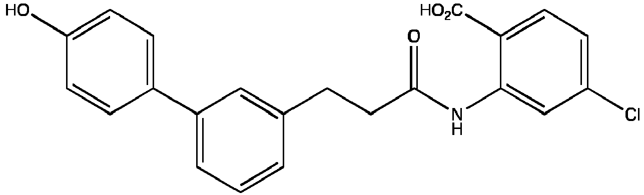 |
| 21 | 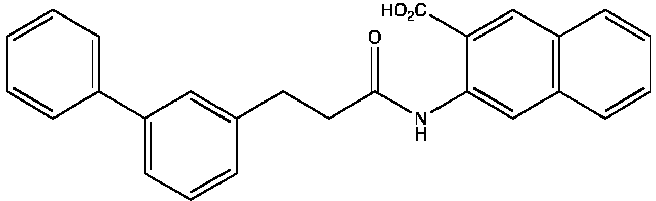 |
| 22 | 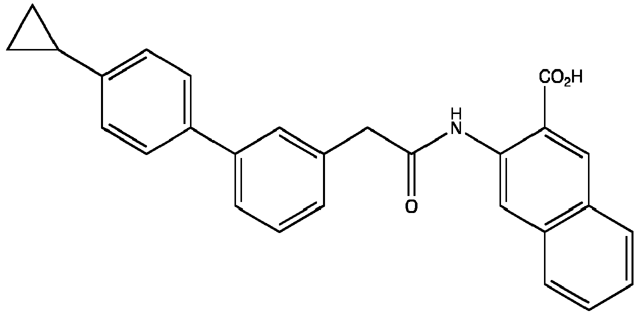 |
| 23 | 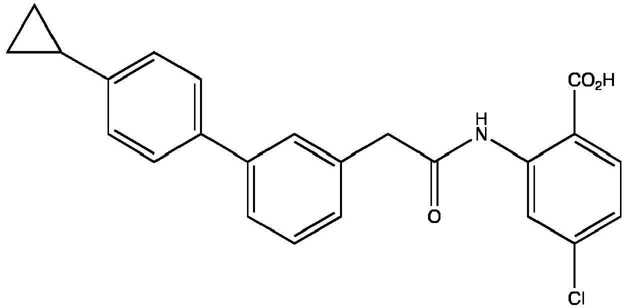 |
| 24 | 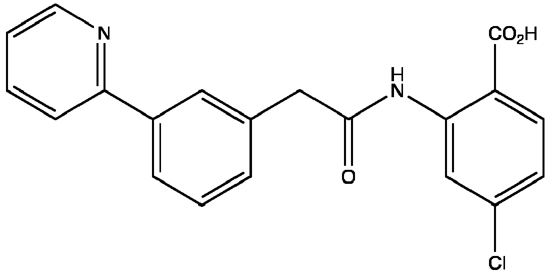 |
| 25 | 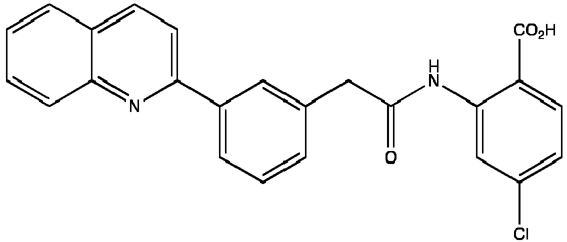 |
| 26 | 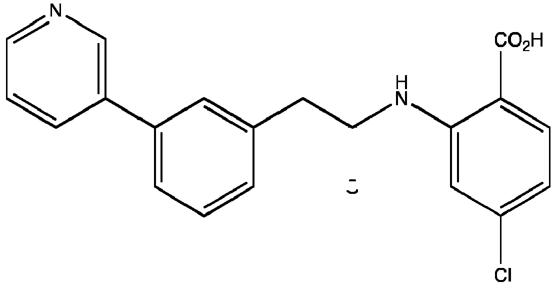 |
| 27 | 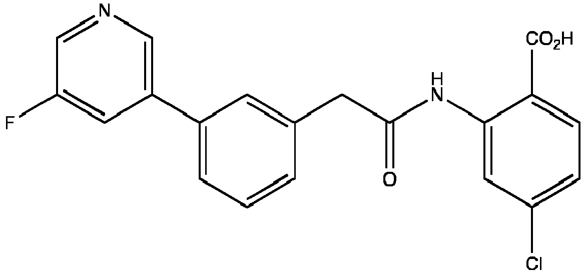 |
| 28 | 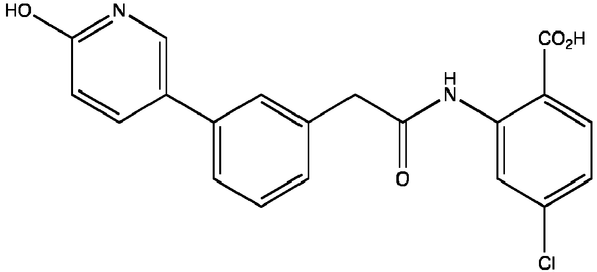 |
| 29 | 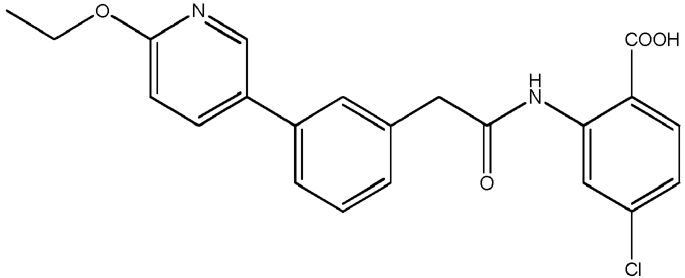 |
| 30 | 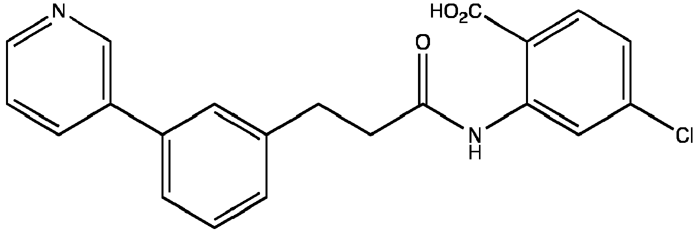 |
| 31 | 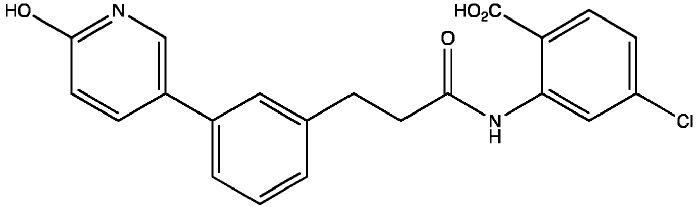 |
| 32 | 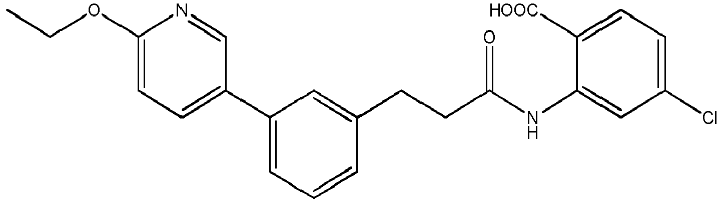 |
| 33 | 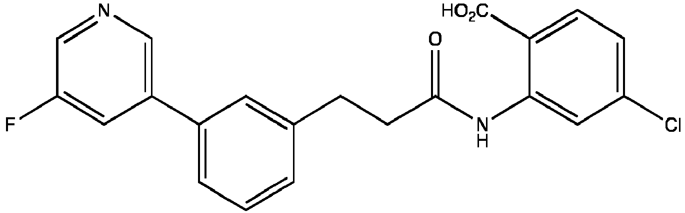 |
| 34 | 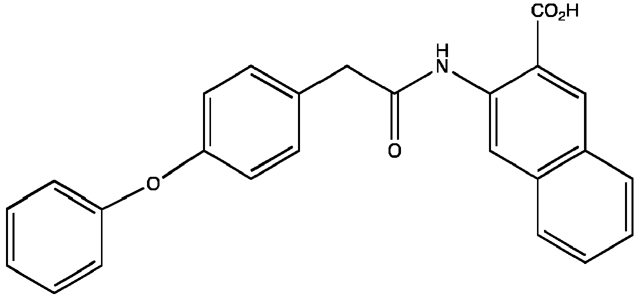 |
| 35 | 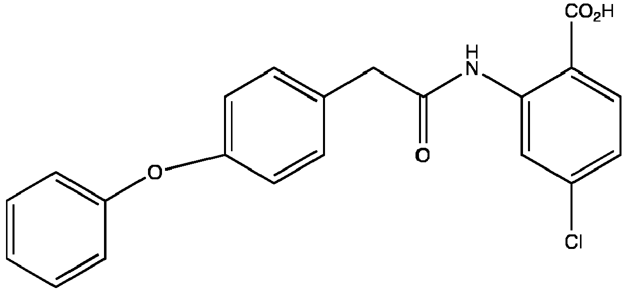 |
| 36 | 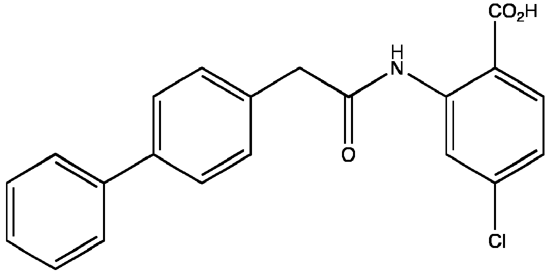 |
| 37 | 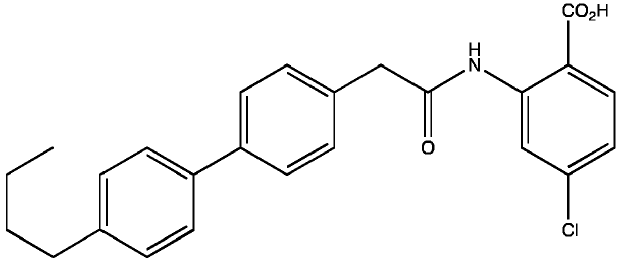 |
| 38 | 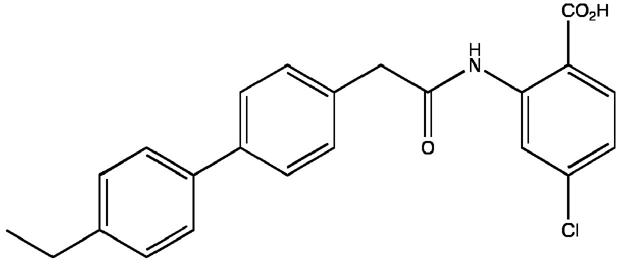 |
| 39 | 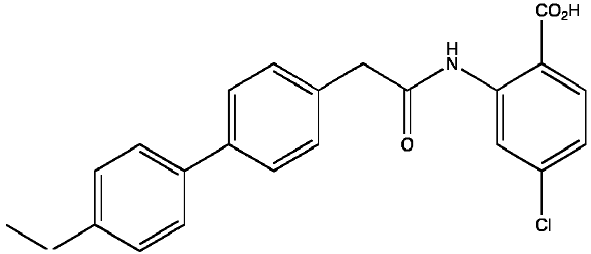 |
| 40 | 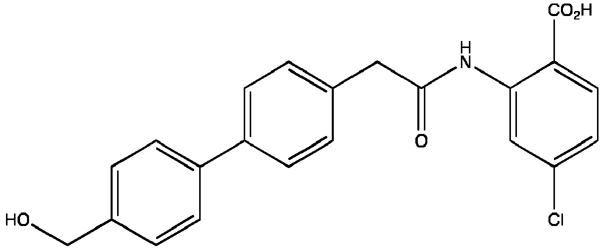 |
| 41 | 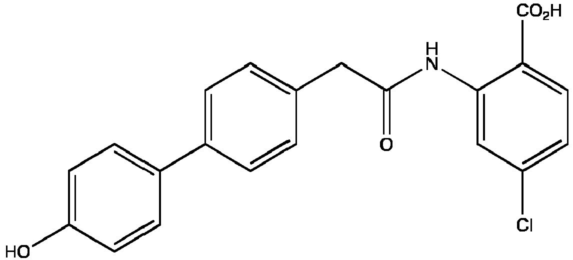 |
| 42 | 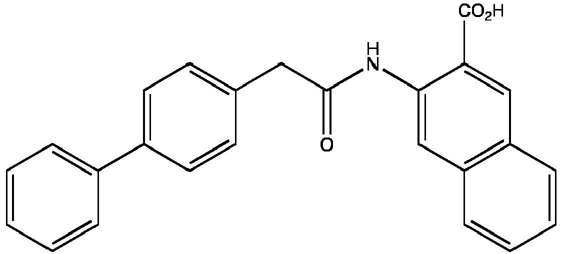 |
| 43 | 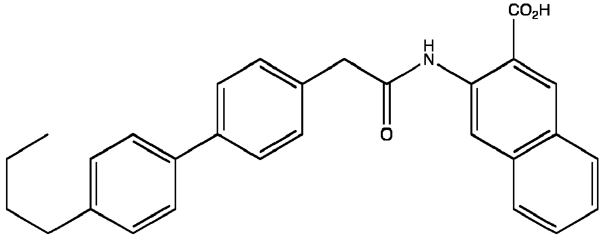 |
| 44 | 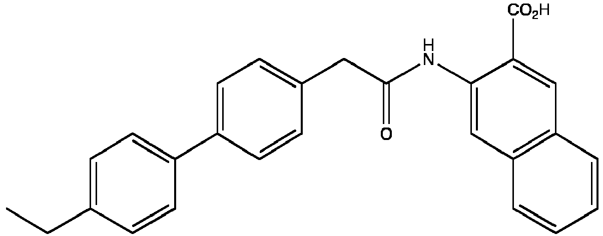 |
| 45 | 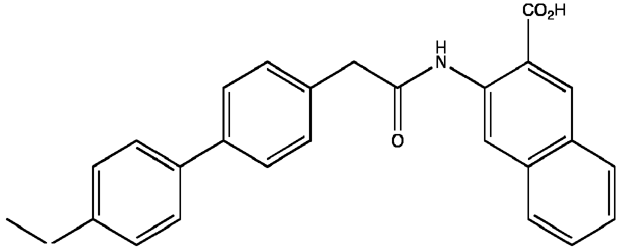 |
| 46 | 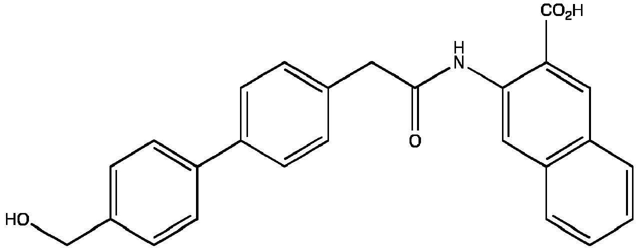 |
| 47 | 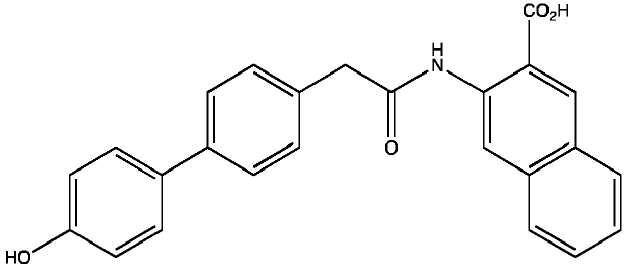 |
| 48 | 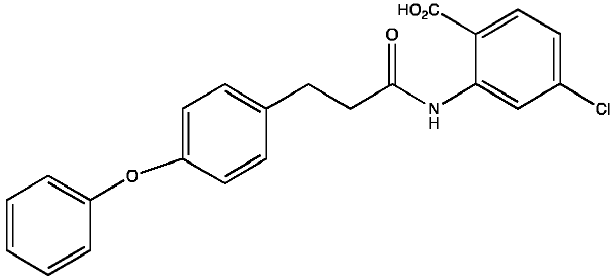 |
| 49 | 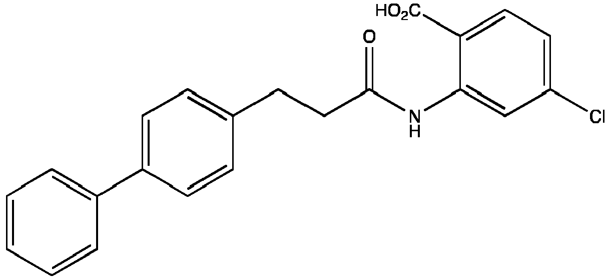 |
| 50 | 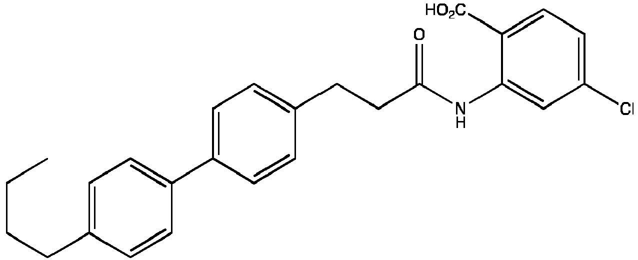 |
| 51 | 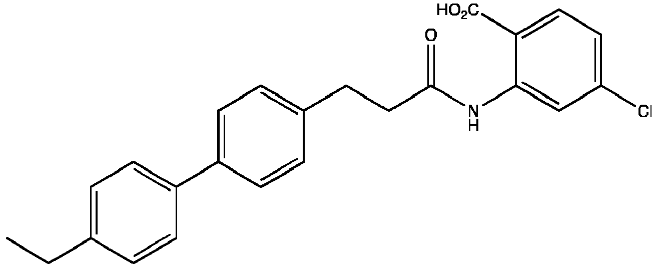 |
| 52 | 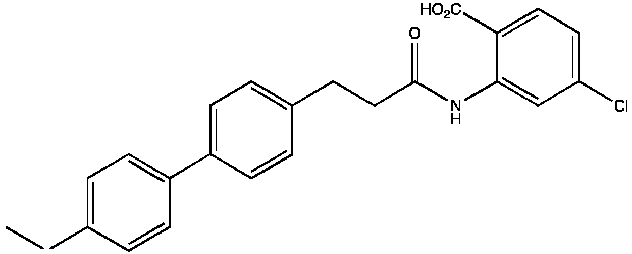 |
| 53 | 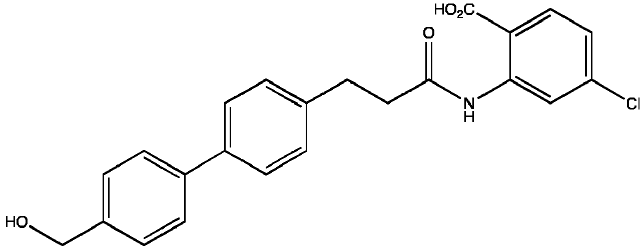 |
| 54 | 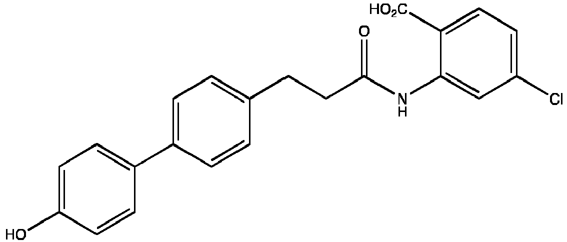 |
| 55 | 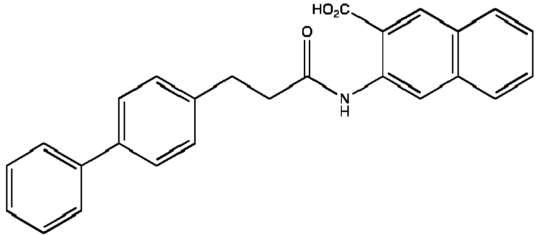 |
| 56 | 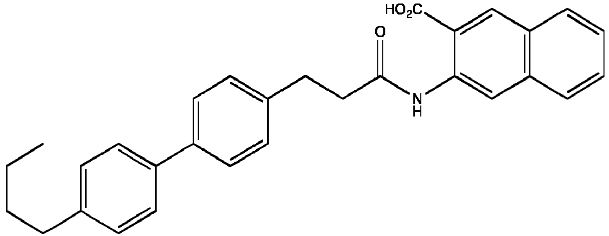 |
| 57 | 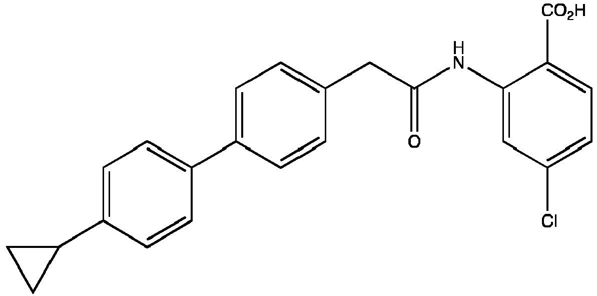 |
| 58 | 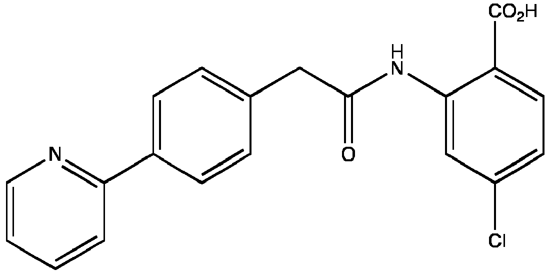 |
| 59 | 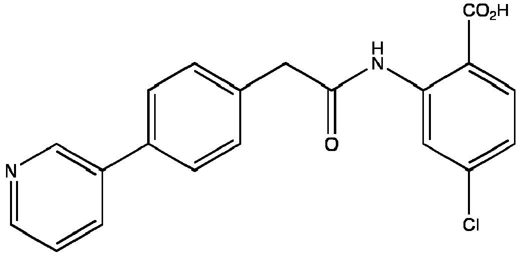 |
| 60 | 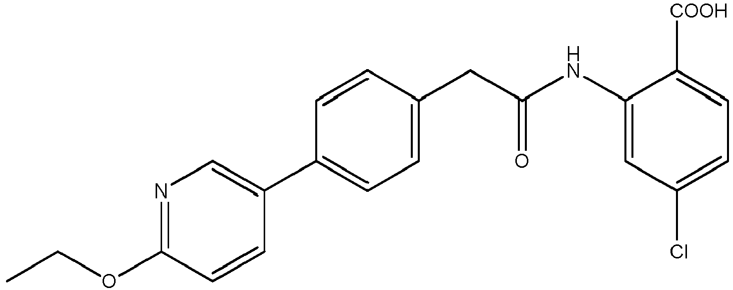 |
| 61 | 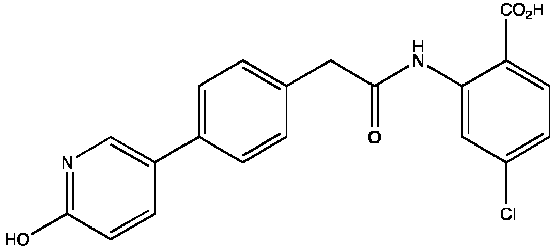 |
| 62 | 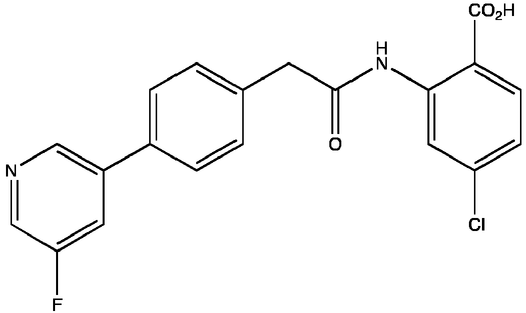 |
| 63 | 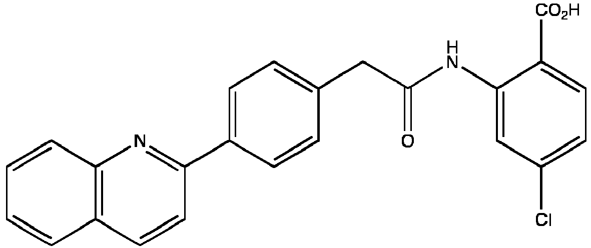 |
| 64 | 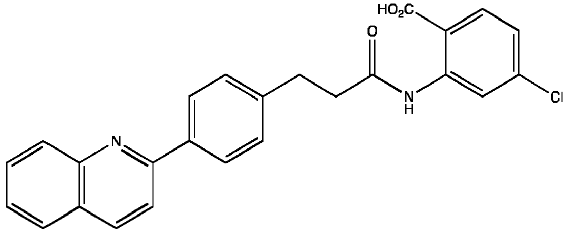 |
| 65 | 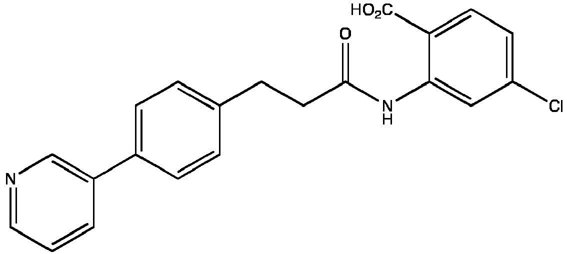 |
| 66 | 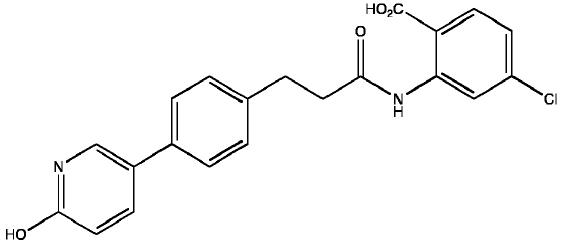 |
| 67 | 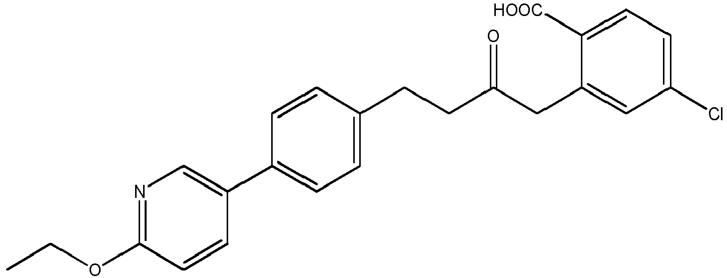 |
| 68 | 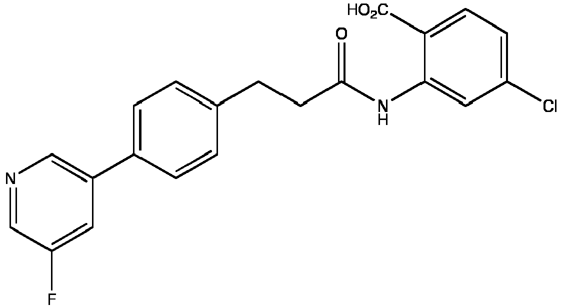 |
| 5bis | 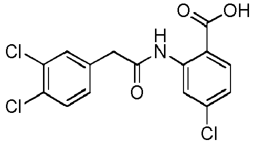 |
| 7bis | 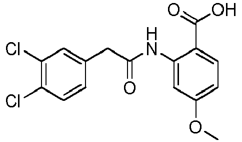 |
| 9bis | 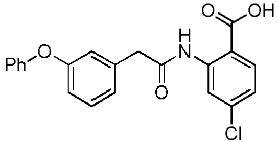 |
| 15bis | 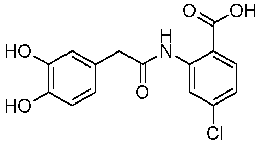 |
| 16bis | 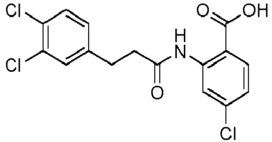 |
| 20bis | 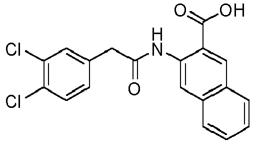 |
| 22bis | 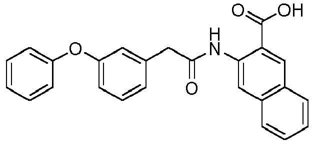 |
| 23bis | 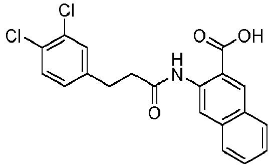 |
| 32bis | 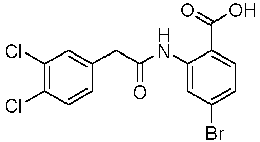 |
| 34bis | 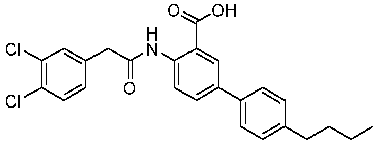 |
| 35bis | 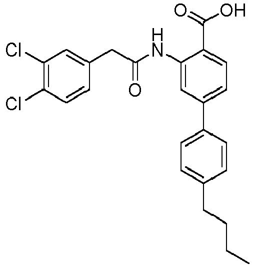 |
| 36bis | 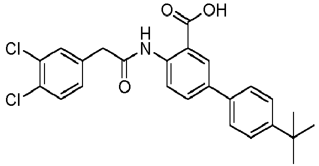 |
| 37bis | 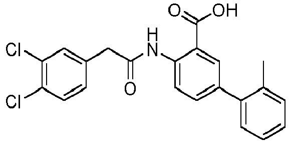 |
| 38bis | 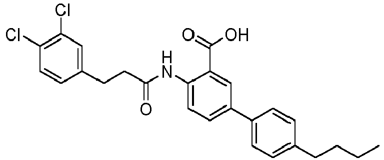 |
| 39bis | 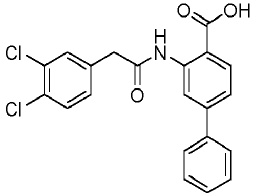 |
| 40bis | 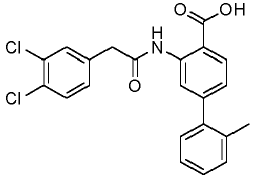 |
| 41bis | 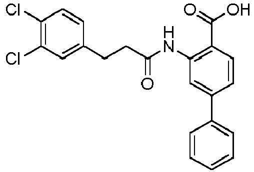 |
| 42bis | 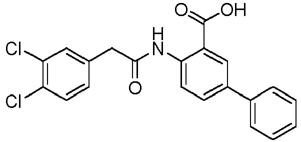 |
| 43bis | 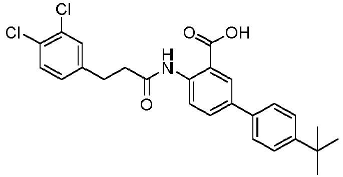 |
| 44bis | 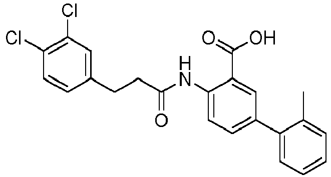 |
| 45bis | 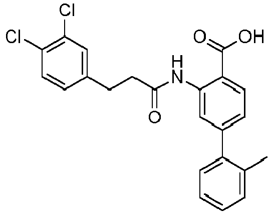 |
| 47bis | 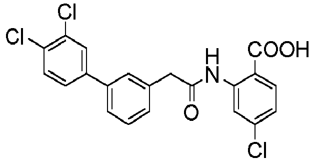 |

Throughout the description and the claims, the word "comprises" and its variants do not intend to exclude other technical features, additives, components or steps. For persons skilled in the art, other objects, advantages and characteristics of the invention may be inferred from both the description and the embodiment of the invention. The following examples and figures are provided by way of example and are not intended to limit the present invention.

### BRIEF DESCRIPTION OF THE FIGURES

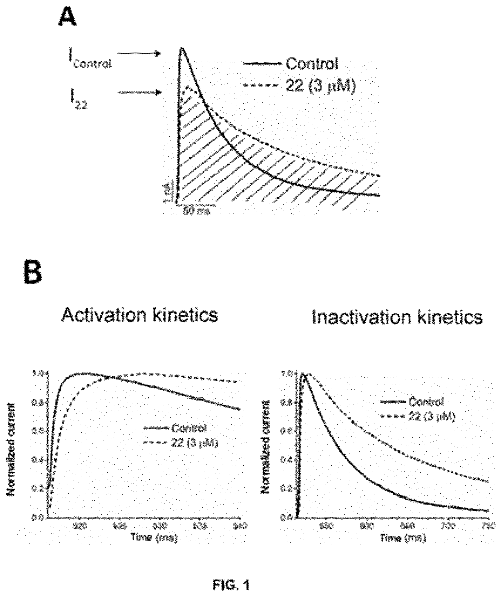

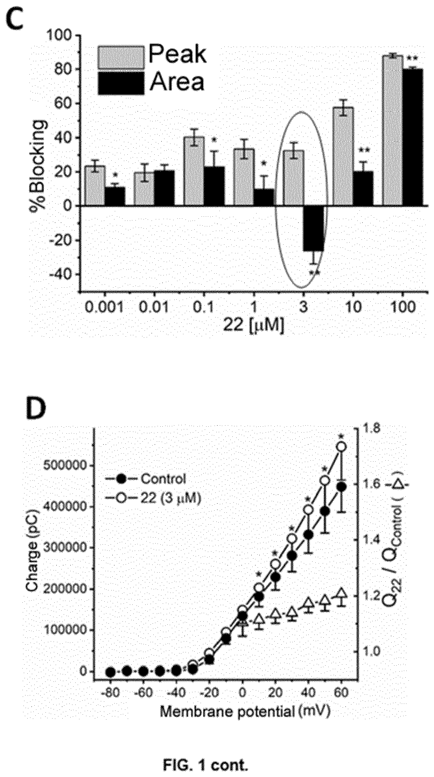

**Fig. 1****. A:** Shows the currents generated by the activation of K_{V}4.3+KChIP2 channels in the absence (continuous line) and in the presence (dotted line) of compound **22** at 3 µM. **B**: Shows the effect of compound **22** on the activation and inactivation kinetics after depolarizing the membrane from -80 mV to +60 mV. **C:** Shows the concentration-dependent effects of compound **22** on changes in the peak and in the charge by means of a bar chart of the blocking produced by different concentrations of compound **22** on the maximum peak of the current and on the charge (measured as the area generated under the current after applying depolarizing pulses at +60 mV) in K_{V}4.3+KChIP2 channels (n = 4-7). p < 0.05 *, p < 0.01 **: statistically significant differences compared to blocking at the peak. **D:** Shows the charge-voltage (Q-V) ratio of K_{V}4.3+KChIP2 in the absence or presence of compound **22** (3 µM). It also shows the voltage-dependence of the increase in the charge produced by **22** expressed as Q_{PC266}/Q_{control}. p<0.05*: statistically significant differences compared to the control.

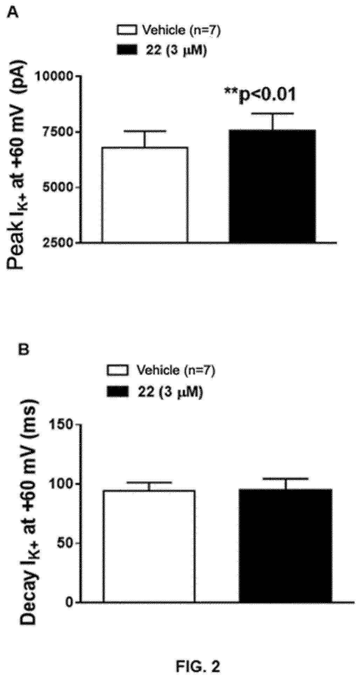

**Fig. 2****.** Shows the mean values of the peak K⁺ current (**A**) and the inactivation time constant (**B**), obtained at +60 mV in seven mouse ventricular cardiomyocytes first perfused with vehicle and then with compound **22** (3 µM) for 3-4 minutes.

### EXAMPLES

### Example 1. Synthesis of the compounds of the invention.

The compounds of general formula (I) of the present invention, can be synthesized in two steps following general methods A-D, depending on the possible substituents. In methods A-C, the first step consists of forming the necessary acid chlorides; and the second step consists of generating the amide, by means of reacting the different acid chlorides and the amine of interest. Method D consists of using peptide coupling agents. These methods are described in detail below:

### Method A

2.4 mmol of oxalyl chloride and a drop of DMF are added, as a catalyst, to a solution of the corresponding carboxylic acid (1 mmol) in anhydrous THF (3 ml) at 0 °C. The reaction mixture is stirred for two hours at room temperature. The acid chloride formed is dissolved in anhydrous THF (3 ml) and the corresponding amine (1.1 mmol) is added. Then, 3 equivalents of anhydrous Et₃N (3 mmol) at 0 °C are added dropwise and it is stirred overnight at room temperature. The solvent is removed under vacuum and the crude reaction product is suspended in water, acidified with 1N HCl to pH=3 or 4, extracted with AcOEt and washed with a saturated solution of NaCl (3 x 15 ml). The organic phase is dried over Na₂SO₄ and concentrated under vacuum. As indicated in each case, the resulting residue is purified by medium-pressure chromatography or by crystallization.

### Method B

The process for synthesizing the acid chloride is the one described in method A. Amide formation is carried out by means of heating in a microwave at 100°C for 5 min using THF as solvent.

### Method C

A solution of the corresponding carboxylic acid (0.75 mmol) in thionyl chloride (1.5 ml) is heated under reflux for 6 h. After this time, the excess thionyl chloride is evaporated to dryness. Then, the residue is dissolved in anhydrous THF (2 ml), and the corresponding amine (0.5 mmol) and propylene oxide (7.5 mmol) are added to the solution. The reaction is stirred at room temperature overnight. Lastly, the excess solvent is removed under vacuum and the solid formed is washed with water. The synthesized produce is purified by successive washes with the suitable solvent or by means of medium-pressure chromatography.

### Method D

2.2 mmol of DIPEA or NMM are added to a solution of the corresponding amine (0.7 mmol) in DMF (2 ml). The solution is stirred at room temperature for 10 min. Then, a coupling agent (1.1 mmol, HATU, COMU, PyAOP-HOAt, EDC, DIC, HOBt) and the corresponding acid (1.1 mmol) are added. After 12 h of stirring at room temperature, the solvent is removed at low pressure. The crude reaction product is suspended in water, acidified with 1N HCl to pH=3 or 4, extracted with AcOEt (3 x 15 ml) and washed with a saturated solution of NaCl (3 x 15 ml). The organic phase is dried over Na₂SO₄ and the solvent is evaporated to dryness. The resulting residue is purified by medium-pressure chromatography.

### Functionalization of the aryl ring. General process.

Cross-coupling technology allows the functionalization of an aryl ring through reactions catalyzed by a transition metal. For example, a Suzuki coupling can be carried out using aryl bromide and a boronic acid coupling partner. Alternatively, couplings between a terminal acetylene and an aryl halide can be carried out by means of the Sonogashira reaction.

### a. Suzuki coupling

An aryl halide (0.4 mmol), the corresponding boronic acid derivative (0.6 mmol), K₂CO₃ (2.6 mmol), [Pd(PPh₃)₄] (2 % by weight) and 7 ml of a THF/H₂O (4/1) mixture are added in a microwave tube. The reaction mixture is purged with argon and heated by irradiating at 125 °C for 15 min in a microwave reactor. Then, an additional 0.6 mmol of the corresponding boronic acid are added and the described process is repeated. The solvent is removed to dryness, water is added and extracted with DCM (3 x 10 ml). The organic phases are washed with H₂O (3x 10 ml), dried over Na₂SO₄, and concentrated at low pressure. The crude reaction product is purified by medium-pressure chromatography.

### b. Sonogashira reaction

The corresponding brominated derivative (0.22 mmol), CuI (0.06 mmol), [Pd(PPh₃)₄] (20 % by weight), Et₃N (1.74 mmol), trimethylsilylalkyne(0,67 mmol) and 1.5 ml of a THF/DMF (10/3) mixture are added in a sealed tube with a 25 ml capacity. The reaction mixture is heated at 45 °C for 12 h. The solvent is evaporated to dryness and the residue is extracted with AcOEt (3 × 10 ml). The organic phases are washed with H₂O (3 × 10 ml), dried over Na₂SO₄, and concentrated at low pressure. The crude reaction product is purified by medium-pressure chromatography (hexane/AcOEt).

### Ester group saponification. General process

A 2N NaOH solution (0.2 ml) is added dropwise to a solution of the corresponding ester (0.09 mmol) in 1.2 ml of THF and 0.6 ml of MeOH. After 12 h of stirring at room temperature, the solvent is removed at low pressure, water is added and it is acidified with 1N HCI to pH 3 or 4. The aqueous phase is extracted with AcOEt (3 x 10 ml). The organic extracts are washed with water and a saturated solution of NaCl, dried over Na₂SO₄, the solvent is removed to dryness and lyophilized. The product is obtained in pure form without requiring additional purifications.

### 2-[2-(1,1'-biphenyl)-3-yl-acetylamino]-4-chlorobenzoic acid (2)

Solid. Yield 66 %. **¹H-NMR** (400 MHz, DMSO-d₆) δ(ppm): 11.37 (s, 1H, NHCO), 8.64 (d, 1H, *J* = 2.2 Hz, H₃), 7.95 (d, 1H, *J* = 8.5 Hz, H₆), 7.63 (m, 4H, H_{2'}, H_{4'}, H_{6'}, H_{2"}), 7.50-7.33 (m, 5H, H₅, H_{3"}, H_{5"}, H_{5'}, H_{6'}), 7.20 (dd, 1H, *J* = 8.6, 2.2 Hz, H_{4"}), 3.87 (s, 2H, CH₂CO). ¹³**C-NMR** (75 MHz, DMSO-d₆) δ (ppm): 170.0 (CONH), 168.7 (COOH), 141.8 (C₂), 140.45 (C₄), 140.0 (C_{1"}), 138.4 (C_{3'}), 135.2 (C_{1'}), 132.8 (C₆), 129.8 (C_{4"}), 128.9 (2C, C_{3"}, C_{5"}), 128.6 (C_{6'}), 128.1 (C_{2'}), 127.5 (C_{5'}), 126.7 (2C, C_{2"}, C_{6"},), 125.4 (C₅), 122.6 (C₄), 119.1 (C₃), 115.2 (C₁), 44.6 (CH₂CO). Agilent **HPLC** (gradient 50-95 % of A in B, 20min): t_{R} = 8.18 min.

### 2-[2-(4-butyl-1,1'-biphenyl-3-yl)acetylamino]-4-chlorobenzoic acid (3)

White syrup. Yield 42 %. **¹H-NMR** (400 MHz, DMSO-d₆) δ(ppm): 11.31 (s, 1H, NHCO), 8.64 (d, 1H, *J* = 2.2 Hz, H₃), 7.95 (d, 1H, *J* = 8.5 Hz, H_{2'}), 7.73 - 7.50 (m, 4H, H₆, H_{6"}, H_{2"}, H_{4'}) 7.48 - 7.15 (m, 5H, H₅, H_{5'}, H_{6'}, H_{3"}, H_{5"}), 3.86 (s, 2H, CH₂CO), 2.60 (t, 2H, *J* = 7.7Hz, 4"1CH₂), 1.65 - 1.50 (m, 2H, 4"2CH₂), 1.32 (h, 2H, *J* = 7.3Hz, 4"3CH₂), 0.90 (t, 3H, *J* = 7.3 Hz, 4"CH₃). **¹³C-NMR** (75 MHz, DMSO-d₆) δ (ppm): 170.0 (CONH), 168.7 (COOH), 141.9 (C₂), 141.7 (C₄), 140.4 (C_{3'}), 138.5 (C_{1"}), 137.3 (C_{4"}), 135.1 (C_{1'}), 132.8 (C₆), 129.2 (C_{6'}), 128.9 (2C, C_{2"}, C_{6"}), 128.3 (C_{2'}), 127.8 (C_{5'}), 126.6 (2C, C_{3"}, C_{5"}), 125.2 (C₅), 122.6 (C_{4'}), 119.1 (C₃), 115.0, (C₁), 44.6 (CH₂CO), 34.4 (4"1 CH₂), 33.1 (4"2CH₂), 21.8 (4"3CH₂), 13.8 (4"CH₃). **LC-MS:** 422.2 ([M+H]⁺).

### 4-chloro-2-(2-(4'-ethyl-[1,1'-biphenyl]-3-yl) acetylamino)benzoic acid (4)

Solid (60 mg). Yield 75 %. HPLC (Sunfire): t_{R}= 2.82 min (gradient of 70 to 95 % of solvent A in B, 10 min). HPLC (Eclipse): t_{R}= 10.49 min (gradient of 50 to 100 % of solvent A in B, 20 min).¹H-NMR (400 MHz, DMSO-*d₆*) δ: 11.86 (br s, 1H, NHCO), 8.61 (d, 1H, *J*=2.2 Hz, H₃), 7.95 (d, 1H, *J*=8.5 Hz, H₆), 7.63 (m, 1H, H_{2'}), 7.59 (d, 2H, *J*=8.2 Hz, H_{2"}, H_{6"}), 7.54 (s, 1H, H_{4'}), 7.42 (t, 1H, *J*=7.6 Hz, H_{5'}), 7.33 (m, 1H, H₆), 7.30 (d, 2H, *J*=8.1 Hz, H_{5"}, H_{3"}), 7.16 (dd, 2H, *J*=8.5, 2.2Hz, H₅), 3.83 (s, 2H, CH₂CO), 2.64 (q, 2H, *J*=7.6 Hz, CH₂), 1.21 (t, 3H, *J*=7.6 Hz, CH_{3,}) ppm. ¹³C-NMR (75 MHz, DMSO-*d₆*) δ: 169.9 (CO₂H), 168.6 (CONH), 143.1 (C₂), 141.8 (C₄), 140.4 (C_{4"}), 137.7 (C_{3'}), 137.4 (C_{1"}), 135.3 (C_{1'}), 132.8 (C₆), 129.1 (C_{6'}), 128.3 (3C, C_{2",} C_{6",} C_{2'}), 128.2 (C₅), 127.7 (C_{5'}), 126.6 (2C, C_{3",} C_{5"}), 125.1 (C₄), 122.3 (C₃), 118.8 (C₁), 44.6 (CH₂CO), 27.8 (CH₂), 15.6 (CH₃) ppm. EM (ES-): m/z 392.27 (M-H)+. Chemical Formula: C₂₃H₂₀ClNO₃. Elemental Analysis Calculated: C, 70.14; H, 5.12; Cl, 9.00; N, 3.56; O, 12.19.

### 2-(2-(4'-(tert-butyl)-[1,1'-biphenyl]-3-yl)acetylamino)-4-chlorobenzoic acid (5)

Solid (66 mg). Yield 66 %. HPLC (Sunfire): t_{R}= 7.14 min (gradient of 60 to 95 % of solvent A in B, 10 min). HPLC (Eclipse): t_{R}= 12.18 min (gradient of 50 to 100 % of solvent A in B, 20 min).¹H-NMR (400 MHz, DMSO-*d₆*) δ: 11.37 (br s, 1H, NHCO), 8.64 (d, 1H, *J*=2.2 Hz, H₃), 7.95 (d, 1H, *J*=8.6 Hz, H₆), 7.64 (s, 1H, H_{2'}), 7.60 (d, 2H, *J*=8.4 Hz, H_{6"}, H_{2"}), 7.56 (m, 1H, H_{4'}), 7.48 (d, 2H, *J*=8.4 Hz, H_{3"}, H_{5"}), 7.43 (t, 1H, *J*=7.6 Hz, H_{5'}), 7.32 (m, 1H, H₆), 7.20 (dd, 1H, *J*=8.6, 2.2 Hz, H₅), 3.86 (s, 2H, CH₂CO), 1.31 (s, 9H, CH₃, ^{t}Bu) ppm. ¹³C-NMR (75 MHz, DMSO-*d₆*) δ: 170.0 (CO₂H), 168.7 (CONH), 149.9 (C_{4"}), 141.8 (C₂), 140.3 (C₄), 138.4 (C_{3'}), 137.1 (C_{1"}), 135.4 (C_{1'}), 132.8 (C₆), 129.1 (C_{6'}), 128.3 (C₅), 127.8 (C_{5'}), 126.4 (2C, C_{2"}, C_{6"}), 125.7 (2C, C_{3"}, C_{5"}), 125.2 (C_{2'}), 122.6 (C₄), 119.1 (C₃), 115.2 (C₁), 44.6 (CH₂CO), 34.2 (C, ^{t}Bu), 31.1 (CH₃, ^{t}Bu) ppm. EM (ES+): m/z 422.34 (M+H)+. Chemical Formula: C₂₅H₂₄ClNO₃. Elemental Analysis Calculated: C, 71.17; H, 5.73; Cl, 8.40; N, 3.32; O, 11.38. Elemental Analysis Found: C, 70.99; H, 5.77; N, 3.59.

### 5-chloro-2-(3-(3-phenoxyphenyl)propylamino)benzoic acid (14)

Solid (106 mg). Yield 82 %. HPLC (Sunfire): t_{R}= 6.09 min (gradient of 50 to 95 % of solvent A in B, 10 min). HPLC (Eclipse): t_{R}= 8.96 min (gradient of 50 to 100 % of solvent A in B, 20 min). ¹H-NMR (400 MHz, DMSO-*d₆*) δ: 11.27 (br s, 1H, NHCO), 8.58 (d, 1H, *J*=2.2 Hz, H₃), 7.97 (d, 1H, *J*=8.6 Hz, H₆), 7.31 (m, 5H, H_{5,} H_{6',} H_{3",} H_{4",} H_{5"}), 7.21 (dd, 1H, *J*=8.6, 2.2 Hz, H_{2"}), 7.10 (t, 1H, *J*=7.4 Hz, H_{4'}), 6.96 (m, 3H, H_{3",} H_{6",} H_{5'}), 6.81 (ddd, 1H, *J*=8.1, 2.1 Hz, H_{2'}), 2.93 (t, 2H, *J*=7.4 Hz, CH₂), 2.73 (t, 2H, *J*=7.4 Hz, CH₂) ppm. ¹³C-NMR (75 MHz, DMSO-*d₆*) δ: 170.8 (CO₂H), 168.7 (CONH), 156.7 (2C, C_{3',} C_{1"}), 142.9 (C₂), 141.8 (C₄), 138.3 (C_{1'}), 132.8 (C₆), 130.0 (3C, C_{3"}, C_{5",} C_{5'}), 123.5 (C₅), 123.3 (C_{4"}), 122.5 (C_{6'}), 119.1 (C₃), 118.6 (C₄), 118.5 (2C, C_{2"}, C_{6"}), 116.4 (C_{2'}), 115.2 (C₁), 30.3 (2C, CH₂CH₂CO, CH₂CO) ppm. EM (ES+): m/z 396.17

### 2-[2-(4-phenoxyphenyl)acetylamino]-4-chloro benzoic acid (35)

White solid. Yield 47 **%.¹H-NMR** (400 MHz, DMSO-d6) δ(ppm): 11.27 (s, 1H, NHCO), 8.64 (d, 1H, *J* = 2.1 Hz, H₃), 7.96 (d, 1H, *J* = 8.6 Hz, H₆), 7.38 (m, 4H, H₅, H_{2'}, H_{6'}, H_{5"}), 7.20 (dd, 1H, *J* = 8.5, 2.2 Hz, H_{3"}), 7.15 - 7.10 (m, 1H, H_{4"}), 7.03 - 6.97 (m, 4H, H_{5'}, H_{3'}, H_{2"}, H_{6"}), 3.78 (s, 2H, CH₂CO). ¹³**C-NMR** 100 MHz, DMSO-d6) δ (ppm): 170.1 (CONH), 168.6 (COOH), 156.8 (C_{4'}), 155.79 (C_{1"}), 141.8 (C₂), 138.4 (C₄), 132.8 (C₆), 131.3 (2C, C_{5"}, C_{3"}), 130.0 (2C, C_{2'}, C_{6'}), 129.6 (C_{1'}), 123.3 (C₅), 122.6 (C_{4"}), 119.0 (C₃), 118.9 (2C, C_{6"}, C_{2"}), 118.4 (2C, C_{3'}, C_{5'}), 115.1 (C₁), 43.7 (CH₂CO). **HPLC** (gradient of 50-95 % of A in B, 10 min): t_{R} = 5.63 min. **LC-MS:** 382.2 ([M+H]⁺).
(M+H)+.

### 2-(2-(4'-(tert-butyl)-[1,1'-biphenyl]-4-yl)acetylamino)-4-chlorobenzoic acid (39)

Solid (12 mg). Yield 29 %. HPLC (Sunfire): t_{R}= 7.02 min (gradient of 60 to 95 % of solvent A in B, 10 min). HPLC (Eclipse): t_{R}= 12.40 min (gradient of 50 to 100 % of solvent A in B, 20 min).¹H-NMR (400 MHz, DMSO-*d₆*) δ: ¹H-NMR (400 MHz, DMSO-*d₆*) δ: 11.47 (br s, 1H, NHCO), 8.62 (d, 1H, *J*=2.2 Hz, H₃), 7.96 (d, 1H, *J*=8.5 Hz, H₆), 7.63 (d, 2H,, *J*=8.2 Hz, H_{2'}, H₆), 7.59 (d, 2H,, *J*=8.5Hz, H_{2"}, H_{6"}), 7.47 (d, 2H,, *J*=8.4 Hz, H_{3"}, H_{5"}), 7.42 (d, 2H,, *J*=8.2 Hz, H_{3'}, H_{5'}), 7.20 (dd, 2H, *J*=8.6, 2.2 Hz, H₅), 3.81 (s, 2H, CH₂CO), 1.31 (t, 3H, *J*=7.6 Hz, CH₃,) ppm. ¹³C-NMR (75 MHz, DMSO-*d₆*) δ: 170.0 (CO₂H), 168.7 (CONH), 149.8 (C4"), 141.8 (C₂), 138.7 (C₄), 138.2 (C₄), 136.9 (C_{1"}), 133.5 (C_{1'}), 132.8 (C₆), 130.1 (2C, C_{2",} C_{6",}), 126.6 (2C, C_{3',} C_{5'}), 126.2 (2C, C_{2',} C_{6'}), 125.7 (2C, C_{3"}, C_{5"}), 122.6 (C₅), 119.0 (C₃), 115.5 (C₁), 44.2 (CH₂CO), 34.2 (C, ^{t}Bu), 31.3 (CH₃, ^{t}Bu) ppm. EM (ES-): m/z 420.35 (M-H)+.

### 4-chloro-2-(2-(3',4'-dichloro-[1,1'-biphenyl]-3-yl) acetylamino)benzoic acid (47bis)

Solid (19.5 mg). Yield 47%. HPLC (Sunfire): t_{R}= 8.54 min (gradient of 50 to 95 % of solvent A in B, 10 min). HPLC (Eclipse): t_{R}= 17.66 min (gradient of 5 to 100 % of solvent A in B, 20 min).¹H-NMR (400 MHz, DMSO-*d₆*) δ: 11.42 (br s, 1H, NHCO), 8.63 (d, 1H, *J*=2.2 Hz, H₃), 7.95 (d, 1H, *J*=8.4 Hz, H₆), 7.93 (s, 1H, H_{2'}), 7.73 (s, 1H, H_{2"}), 7.70 (d, 1H, *J*=4.1 Hz, H_{4'}), 7.69 (m, 1H, H₆), 7.64 (dd, 1H, *J*=7.7, 1.6 Hz, H_{6"}), 7.45 (t, 1H, *J*=7.6 Hz, H_{5'}), 7.40 (d, 1H, *J*=7.7 Hz, H_{5"}), 7.19 (dd, 1H, *J*=8.6, 2.2 Hz, H₅), 3.87 (s, 2H, CH₂CO) ppm. ¹³C-NMR (75 MHz, DMSO-*d₆*) δ: 169.8 (CO₂H), 168.6 (CONH), 141.8 (C₂), 140.6 (C₄), 138.2 (C_{3'}), 137.7 (C_{1"}), 135.4 (C_{1'}), 132.8 (C₆), 131.7 (C_{3"}), 131.0 (C_{6'}), 130.2 (C_{6"}), 129.6 (C_{2"}), 129.3 (C_{4"}), 128.5 (C_{2'}), 128.2 (C_{5"}), 126.9 (C_{5'}), 125.5 (C₅), 122.6 (C₄), 119.0 (C₃), 115.5 (C₁), 44.3 (CH₂CO) ppm. EM (ES-): m/z 432.99 (M-H)+.

### Example 2. Tests of the effects of the compounds on the K_{V}4.3/KChIP2 current generated after the activation of K_{V}4.3/KChIP2 channels expressed in CHO cells.

The compounds synthesized according to this invention have been evaluated *in vitro* in voltage clamp tests in CHO cells transiently transfected with cDNA encoding K_{V}4.3 alone or K_{V}4.3 in the presence of KChIP2 (K_{V}4.3+KChIP2) using the whole-cell configuration of the patch-clamp technique. Some of the compounds activate the current generated by the activation of K_{V}4.3+KChIP2 channels. Thus, as observed in Fig. 1A, compound 22 lowers the maximum peak, but increases the charge measured as the area under the current record calculated from the current integral. Fig. 1B shows the effect of 22 on the activation and inactivation kinetics after depolarizing the membrane from -80 mV to +60 mV. As it can be observed, this compound delays the kinetics of both processes. Fig. 1C shows the concentration-dependent effects of compound 22 on changes in the peak and in the charge. At all the concentrations tested (except 0.01 µM), 22 produced a decrease in the peak that was significantly greater than that of the charge. At the concentration of 3 µM (indicated in the figure with a red circle), compound 22 produced an increase in the charge. Fig. 1D shows the charge-voltage relationship (Q-V). Compound 22 produced an increase in the charge of the K_{V}4.3/KChIP2 current which turned out to be significant at potentials positive than 0 mV. This result is relevant, given that membrane potentials comprised between -10 and +30 mV are physiological.

### Example 3. Effects of the compounds on the K⁺ current recorded in mouse ventricular cardiomyocytes.

Fig. 2 shows the effects of compound 22 (3 µM) on the peak (A) and on the decay time constant (B) of the K⁺ current obtained at +60 mV in ventricular cardiomyocytes obtained from C57BL/6J mice (3-4 months of age) using the perforated patch configuration of the patch-clamp technique. The cardiomyocytes were obtained after enzyme dissociation with collagenase. The bar graph shows that perfusion with compound 22 (3 µM) for 3-4 minutes produced a significant increase in the maximum peak current without modifying the decay time constant.

## Claims

1. A compound of formula (I): 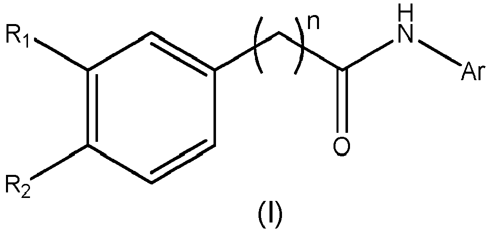 wherein:
R₁ and R₂ are independently selected from H, OH, -O-aryl, halogen, aryl or heteroaryl, wherein said aryl or heteroaryl groups can be optionally substituted with (C₁-C₆) alkyl, - OH, (C₃-C₆) cycloalkyl, -O-(C₁-C₆) alkyl or halogen;
n has a value of 1 or 2;
Ar is selected from the following groups: 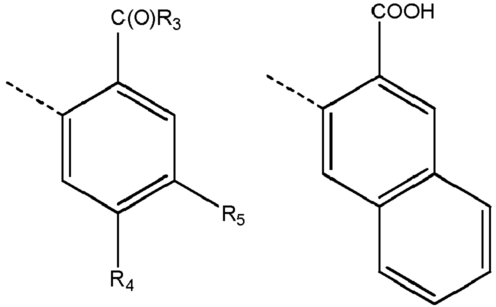
wherein R₃ is selected from -OH or -O-(C₁-C₆)-alkyl; R₄ is selected from H, halogen, -O-(C₁-C₆)-alkyl, phenyl optionally substituted with (C₁-C₆) alkyl; R₅ is selected from H or phenyl optionally substituted with alkyl (C₁-C₆), or the salts, isomers or solvates thereof, for use in the treatment of heart diseases which are selected from heart arrhythmia myocardial ischemia, myocardial infarction, cardiac hypertrophy or cardiomyopathy.

2. The compound for use according to claim 1 which is the compound of formula (Ia): 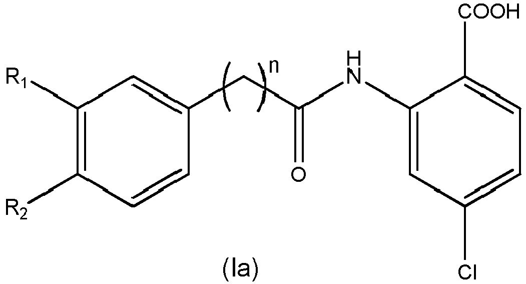 wherein R₁, R₂ and n are defined as in claim 1, or the salts, isomers thereof.

3. The compound for use according to claim 2, wherein R₁ is selected independently from O-phenyl, phenyl, quinoline or pyridine, wherein said phenyl is optionally substituted with a group which is selected from (C₁-C₄) alkyl, -OH or (C₃) cycloalkyl and said pyridine is optionally substituted with a group which is selected from -O-(C₁-C₄) alkyl, -OH or halogen and R₂ is H.

4. The compound for use according to any of claims 2 or 3, said compound being selected from the following list:
• 4-chloro-2-(2-(3-phenoxyphenyl)acetamido)benzoic acid (1)
• 2-(2-([1,1'-biphenyl]-3-yl)acetamido)-4-chlorobenzoic acid (2)
• 2-(2-(4'-butyl-[1,1'-biphenyl]-3-yl)acetamido)-4-chlorobenzoic acid (3)
• 4-chloro-2-(2-(4'-ethyl-[1,1'-biphenyl]-3-yl)acetamido)benzoic acid (4)
• 2-(2-(4'-(*tert*-butyl)-[1,1'-biphenyl]-3-yl)acetamido)-4-chlorobenzoic acid (5)
• 4-chloro-2-(2-(4'-hydroxy-[1,1'-biphenyl]-3-yl)acetamido)benzoic acid (6)
• 4-chloro-2-(2-(4'-(hydroxymethyl)-[1,1'-biphenyl]-3-yl)acetamido)benzoic acid (7)
• 4-chloro-2-(3-(3-phenoxyphenyl)propanamido)benzoic acid (14)
• 2-(3-([1,1'-biphenyl]-3-yl)propanamido)-4-chlorobenzoic acid (15)
• 2-(3-(4'-butyl-[1,1'-biphenyl]-3-yl)propanamido)-4-chlorobenzoic acid (16)
• 4-chloro-2-(3-(4'-ethyl-[1,1'-biphenyl]-3-yl)propanamido)benzoic acid (17)
• 2-(3-(4'-(*tert*-butyl)-[1,1'-biphenyl]-3-yl)propanamido)-4-chlorobenzoic acid (18)
• 4-chloro-2-(3-(4'-(hydroxymethyl)-[1,1'-biphenyl]-3-yl)propanamido)benzoic acid (19)
• 4-chloro-2-(3-(4'-hydroxy-[1,1'-biphenyl]-3-yl)propanamido)benzoic acid (20)
• 4-chloro-2-(2-(4'-cyclopropyl-[1,1'-biphenyl]-3-yl)acetamido)benzoic acid (23)
• 4-chloro-2-(2-(3-(pyridin-2-yl)phenyl)acetamido)benzoic acid (24)
• 4-chloro-2-(2-(3-(quinolin-2-yl)phenyl)acetamido)benzoic acid (25)
• 4-chloro-2-(2-(3-(pyridin-3-yl)phenyl)acetamido)benzoic acid (26)
• 4-chloro-2-(2-(3-(5-fluoropyridin-3-yl)phenyl)acetamido)benzoic acid (27)
• 4-chloro-2-(2-(3-(6-hydroxypyridin-3-yl)phenyl)acetamido)benzoic acid (28)
• 4-chloro-2-(2-(3-(6-ethoxypyridin-3-yl)phenyl)acetamido)benzoic acid (29)
• 4-chloro-2-(3-(3-(pyridin-3-yl)phenyl)propanamido)benzoic acid (30)
• 4-chloro-2-(3-(3-(6-hydroxypyridin-3-yl)phenyl)propanamido)benzoic acid (31)
• 4-chloro-2-(2-(3-(6-ethoxypyridin-3-yl)phenyl)propanamido)benzoic acid (32)
• 4-chloro-2-(3-(3-(5-fluoropyridin-3-yl)phenyl)propanamido)benzoic acid (33)
• 4-chloro-2-[2-(3-phenoxyphenyl)acetylamino]benzoic acid (9bis)
• 4-chloro-2-(2-(3',4'-dichloro-[1,1'-biphenyl]-3-yl) acetylamino)benzoic acid (47bis)

5. The compound for use according to claim 2, wherein R₁ and R₂ are chlorine.

6. The compound for use according to claim 2, wherein R₁ and R₂ are -OH.

7. The compound for use according to claim 1 which is the compound of formula (Ib): 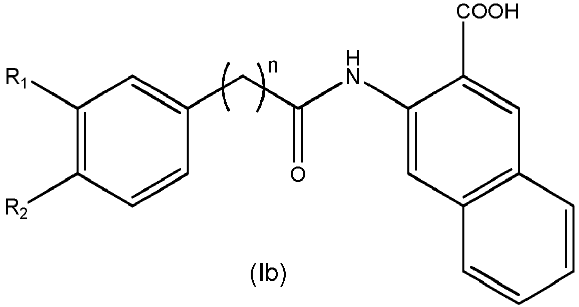 wherein R₁, R₂ and n are defined as in claim 1, or the salts, isomers or solvates thereof.

8. The compound for use according to claim 7, wherein R₁ and R₂ are independently selected from O-phenyl, chlorine, phenyl, quinoline or pyridine, wherein said phenyl is optionally substituted with a group which is selected from (C₁-C₄) alkyl, - OH or (C₃) cycloalkyl and said pyridine is optionally substituted with a group which is selected from -O-(C₁-C₄) alkyl, -OH or halogen and R₂ is H.

9. The compound for use according to any of claims 7 or 8, which is selected from the following list:
• 3-(2-([1,1'-biphenyl]-3-yl)acetamido)-2-naphthoic acid (8)
• 3-(2-(4'-butyl-[1,1'-biphenyl]-3-yl)acetamido)-2-naphthoic acid (9)
• 3-(2-(4'-ethyl-[1,1'-biphenyl]-3-yl)acetamido)-2-naphthoic acid (10)
• 3-(2-(4'-(*tert*-butyl)-[1,1'-biphenyl]-3-yl)acetamido)-2-naphthoic acid (11)
• 3-(2-(4'-hydroxymethyl-[1,1'-biphenyl]-3-yl)acetamido)-2-naphthoic acid (12)
• 3-(2-(4'-(hydroxymethyl)-[1,1'-biphenyl]-3-yl)acetamido)-2-naphthoic acid (13)
• 3-(3-([1,1'-biphenyl]-3-yl)propanamido)-2-naphthoic acid (21)
• 3-(2-(4'-cyclopropyl-[1,1'-biphenyl]-3-yl)acetamido)-2-naphthoic acid (22)
• 3-[2-(3-phenoxyphenyl)acetylamino]-2-naphthoic acid (22bis)

10. The compound for use according to claim 9, wherein R₁ and R₂ are chlorine.

11. The compound for use according to any of claims 7 or 10, said compound being selected from the following list:
• 3-[2-(3,4-dichlorophenyl)acetylamino]-2-naphthoic acid, (20)
• 3-[3-(3-(3,4-dichlorophenyl)propanoylamino)]-2-naphthoic acid, (23)

12. The compound for use according to claim 1, which is a compound of formula (Ic): 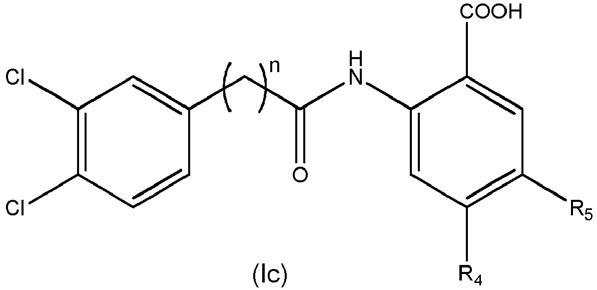 wherein R₃, R₄ and n are defined as in claim 1, or the salts, isomers or solvates thereof.

13. The compound for use according to claim 12, wherein R₄ and R₅ are independently selected from H, O-(C₁-C₄) alkyl or phenyl optionally substituted with a (C₁-C₄) alkyl.

14. The compound for use according to any of claims 12 or 13, said compound being selected from the following list:
• 2-[2-(3,4-dichlorophenyl)acetylamino]- 4-methoxybenzoic acid (7bis)
• 2-[2-(3,4-dichlorophenyl)acetylamino]-5-(4'-n-butylphenyl)benzoic acid (34bis)
• 2-[2-(3,4-dichlorophenyl)acetylamino]-4-(4'-n-butylphenyl)-benzoic acid (35bis)
• 2-[2-(3,4-dichlorophenyl)acetylamino]-5-(4'-*tert*-butylphenyl)-benzoic acid (36bis)
• 2-[2-(3,4-dichlorophenyl)acetylamino]-5-(2'-methylphenyl)-benzoic acid (37bis)
• 2-[3-(3,4-dichlorophenyl)propanoylamino]-5-(4'-n-butylphenyl)benzoic acid (38bis)
• 2-[2-(3,4-dichlorophenyl)acetylamino]-4-phenylbenzoic acid (39bis)
• 2-[2-(3,4-dichlorophenyl)acetylamino]-4-(2'-methylphenyl)benzoic acid (40bis)
• 2-[3-(3,4-dichlorophenyl)propanoylamino]-4-phenylbenzoic acid (41bis)
• 2-[2-(3,4-dichlorophenyl)acetylamino]-5-phenylbenzoic acid (42bis)
• 2-[3-(3,4-dichlorophenyl)propanoylamino]-5-(4'-*tert*-butylphenyl)benzoic acid (43bis)
• 2-[3-(3,4-dichlorophenyl)propanoylamino]-5-(2'-methylphenyl)benzoic acid (44bis)
• 2-[3-(3,4-dichlorophenyl)propanoylamino]-4-(2'-methylphenyl)benzoic acid (45bis)
